# EUROPEAN PATENT APPLICATION

(11) **EP 2 181 717 A1**
(43) Date of publication of application: **05.05.2010**
(21) Application number: 08291013.4
(22) Date of filing: 28.10.2008
(51) Int. Cl.: A61K 51/04, G01N 33/60

(54) **Use of 7-chloro-N,N,5-trimethyl-4-oxo-3-phenyl-3,5-dihydro-4H-pyridazino[4,5-B]indole-1-acetamide as a biomarker of peripheral benzodiazepine receptor levels**

(71) Applicant: Sanofi-Aventis, 75013 Paris (FR); Commissariat à l'Energie Atomique, 91191 Gif sur Yvette Cedex (FR)
(72) Inventor: Benavides, Jésus, 75013 Paris (FR); Castel, Marie-Noëlle, 75013 Paris (FR); Marguet, Frank, 75013 Paris (FR); Rooney, Thomas, 75013 Paris (FR); Rivron, Luc, 75013 Paris (FR); Dolle, Frédéric, 91940 Gometz-le-Châtel (FR); Hantraye, Philippe, 91420 Morangis (FR); Tavitian, Bertrand, 75015 Paris (FR)
(74) Representative: Kugel, Dominique

(57) **Abstract**

Use of 7-chloro-N,N,5-trimethyl-4-oxo-3-phenyl-3,5-dihydro-4H-pyridazino[4,5-*b*]indole-1-acetamide as a biomarker for the detection, in an individual, of PBR levels associated with normal and pathological conditions.

Method for the detection of PBR levels associated with normal and pathological conditions.

Diagnostic kit.

## Description

The current invention relates to the use of 7-chloro-*N,N*,5-trimethyl-4-oxo-3-phenyl-3,5-dihydro4H-pyridazino[4,5-*b*]indole-1-acetamide as a biomarker for the detection, in an individual, of PBR (peripheral benzodiazepine receptor) levels associated with normal and pathological conditions. The current invention also concerns a method of detection of PBR levels for the above purposes.

Under normal physiological conditions, the PBR, also known as translocator protein 18kDa (TSPO) (Papadopoulos, B. et al. (2006) Trends Pharmacol. Sci. 27: 402-409), is expressed at low levels in the brain, mainly in microglial cells, and is highly expressed in a number of peripheral tissues, such as adrenal glands, pineal gland, salivary glands, gonads, kidney, lung, heart and skeletal muscle (Chen, M-K. and Guilarte, T. (2008) Pharmacology and Therapeutics 118: 1-17; Venneti, S. et al. (2006) Progress in Neurobiol. 80: 308-322). Subcellular localization studies with the reference PBR ligand, [³H]PK11195, have demonstrated that the PBR is located in the outer mitochondria membrane (Anholt, R.R. et al. (1986) J. Biol. Chem. 261: 576-583; Antkiewicz-Michaluk, L. et al. (1988) Mol Pharmacol 34: 272-278). However, immunohistochemical studies have also shown that the PBR is expressed in blood cells (devoid of mitochondria) (Olson, J.M. et al. (1988) Eur. J. Pharmacol. 152: 47-53) and can be localized to the plasma membrane (O'Beirne, G. et al. (1990) Eur. J. Biochem. 188: 131-138; Woods, M.J. et al. (1996) Biochem. Pharmacol. 51: 1283-1292). A nuclear or peri-nuclear localization of the PBR has also been observed in breast cancer (Hardwick, M. et al. (1999) Cancer Res. 59: 831-842), human glioma cells (Brown, R.C. et al. Cancer Lett. 156: 125-132), hepatic tumor cells (Corsi, L. et al. Life Sci. 76: 2523-2533) and glial cells (Kuhlmann, A.C. and Guilarte, T.R. (2000) J. Neurochem. 74: 1694- 1704).

A marked increase in PBR levels is observed after cell injury, inflammation or proliferation and is associated with a number of acute and chronic pathological conditions (Chen, M-K. and Guilarte, T. (2008) Pharmacology and Therapeutics 118: 1-17; Venneti, S. et al. (2006) Progress in Neurobiology 80: 308-322). These include: brain injuries, such as stroke and ischemia-reperfusion injury (Gerhard, A. et al. (2000) Neuroreport 11: 2957-2960; Gerhard, A. et al. (2005) Neuroimage 24: 404-412), traumatic brain injury (Raghavendra, R. et al. (2000) Exp. Neurol. 161: 102-114); brain infections, such as encephalitis (Banati, R.B. et al. (1999) Neurology 53: 2199-2203; Cagin, A. et al. (2001) Brain 124: 2014-2027); neurological diseases, such as multiple sclerosis (Banati, R.B. et al. (2000) Brain 123: 2321-2337), Alzheimer's disease and dementia (Cagnin, A. et al. (2001) Lancet 358: 461-467; Versijpt, J.J. et al. (2003) Eur. Neurol. 50: 39-47), Parkinson's disease (Ouchi, Y. et al. (2005) Ann. Neurol. 57: 168-175; Gerhard, A. et al. (2006) Neurobiol. Dis. 21: 404-412), amyotrophic lateral sclerosis (Turner, M.R. et al. (2004) Neurobiol. Dis. 15: 601-609), cortico-basal degeneration (Gerhard A. et al. (2004) Mov. Disord. 19: 1221-1226; Henkel, K. et al. (2004) Mov. Disord. 19: 817-821), Huntington's disease (Messmer, K. and Reynolds, G.P. (1998) Neurosci. Lett. 241: 53-56; Pavese, N. et al. (2006) Neurology 66: 1638-1643), epilepsy (Sauvageau, A. et al. (2002) Metab. Brain Dis. 17: 3-11) The increased PBR levels in CNS pathologies is mainly observed in microglial cells (Chen, M-K. and Guilarte, T. (2008) Pharmacology and Therapeutics 118: 1-17; Venneti, S. et al. (2006) Progress in Neurobiology 80: 308-322).
Large increases in PBR are also observed in cancer (Cornu, P. et al. (1992) Acta. Neurichir. 119: 146-152; Hardwick, M. et al. (1999) Cancer Res. 59: 831-842; Maaser, K. et al. (2002) Cancer Res. 8: 3205-3209), pulmonary inflammation (Audi, S.H. et al. (2002) Lung. 180: 241-250;Hardwick, M.J. et al. (2005) Mol. Imaging. 4: 432-438), cardiac ischemia (Mazzone, A. et al. (2000) J. Am. Coll. Cardiol. 36: 746-750), renal ischemia (Zhang, K. et al. (2006) J. Am. Coll. Surg. 203: 353-364), rheumatism (fibromyalgia) (Faggioli, P. et al. Rheumatology (Oxford). 43: 1224-1225), sciatic nerve regeneration (Mills, C.D. et al. (2005) Mol. Cell. Neurosci. 30: 228-237), psoriasic arthritis (Guisti, L. et al. (2004) Clin. Biochem. 37: 61-66) and atherosclerosis (Fujimara, Y. et al. (2008) J. Atheroslerosis. 24 April online; Laitinen, I. et al. (2008) Eur. J. Nuc. Med. Mol. Imaging. 19 August online).
By contrast, a decrease in PBR levels is observed in the brain of schizophrenia patients (Kurumaji, A. et al. (1997) J. Neural. Transm. 104: 1361-1370; Wodarz, N. et al. (1998) Psychiatry Res. 14: 363-369) and in rheumatoid arthritis (Bribes, E. et al. (2002) Eur. J. Pharmacol. 452: 111-122) and osteoarthritis (Bazzichi, L. et al. Clin. Biochem. 36: 57-60).

The ability to image PBR levels *in vivo* in brain and other tissues could therefore serve as an important biomarker of disease progression, to determine and evaluate the efficacy of a therapeutic treatment and to evaluate PBR receptor occupancy *in vivo.*

The reference PET PBR ligand, [¹¹C] PK11195, has been used extensively for *in vivo* imaging of PBR levels in a number of neuropathological conditions (Chen, M-K and Guilarte, T. (2008) Pharmacology and Therapeutics 118: 1-17; Venneti, S. et al. (2006) Progress in Neurobiology 80: 308-322). However, [¹¹C] PK11195 shows relatively low brain uptake, high non-specific binding and a poor signal to noise ratio. These properties limit the sensitivity of [¹¹C] PK11195 for PET imaging of PBR levels and occupancy studies in the CNS. The development of improved PBR PET ligands with higher specific binding and greater sensitivity than [¹¹C] PK11195 would therefore provide a major advance for the imaging of PBR levels in brain and other tissues.

Among the compounds described and claimed in the international patent applications WO99/06406 and WO00/44384, a pyridazino[4,5-*b*]indole derivative, 7-chloro-*N,N*,5-triméthyle-4-oxo-3-phenyle-3,5-dihydro-4H-pyridazino[4,5-*b*]indole-1-acetamide, was identified as particularly interesting for use as a PET (or SPECT) ligand for the PBR. This compound has high affinity for the PBR *in vitro* and *in vivo* (Ferzaz, B. et al. (2002) J. Pharm. and Exp. Therap. 301: 1067-1078).

### Summary of Invention

The present invention concerns the use of 7-chloro-N,N,5-trimethyl-4-oxo-3-phenyl-3,5-dihydro-4H-pyridazino[4,5-*b*]indole-1-acetamide as a biomarker for the detection of PBR levels associated with normal conditions and PBR levels associated with pathological conditions.
The present invention also concerns a method for the detection of PBR levels associated with normal conditions and PBR levels associated with pathological conditions using a radiolabelled form of 7-chloro-N,N,5-trimethyl-4-oxo-3-phenyl-3,5-dihydro-4H-pyridazino[4,5-b]indole-1-acetamide.
The present invention also concerns a diagnostic kit for the detection of PBR levels.

### Definitions

For convenient reasons and to facilitate reading, the compound 7-chloro-N,N,5-trimethyl-4-oxo-3-phenyl-3,5-dihydro-4H-pyridazino[4,5-b]indole-1-acetamide has been renamed as "A" in some chapters of the current application.

For the present invention the following words are to be understood accordingly.

-Biomarker: a characteristic that can be objectively measured (ie with acceptable precision and reproducibility) and used as an indicator of a normal physiological or pathological process and to evaluate the action of medical therapies. A biomarker can be a biological, anatomic, physiological, biochemical or molecular parameter that can be detected in tissue or biological fluid.

-Inflammation: response to injury or destruction of tissues. In the periphery, acute inflammation consists of leukocytic infiltrates characterized by polymorphonuclear cells (neutrophils) and chronic inflammation consists of mononuclear cells (macrophages, lymphocytes, plasma cells). In the brain, inflammation incorporates a wide spectrum of cellular responses that include activation of microglia and astrocytes and elaboration of cytokines and chemokines, complement proteins, acute phase proteins, oxidative injury, and related molecular processes. These events may have detrimental effects on neuronal function, leading to neuronal injury and further glial activation and ultimately neurodegeneration. In response to brain inflammation, glial cells (mostly microglia) are activated and overexpress the PBR. The levels of PBR in the brain are therefore an indicator of neuroinflammation and can be considered as a biomarker of neuroinflammation for the present invention.

-Pathological conditions: these conditions can include neurological diseases: any injury, disorder or disease that affects the function of the central nervous system. These can include acute brain injuries, such as stroke, ischemia-reperfusion injury, traumatic brain injury; brain infections, such as encephalitis; neurological diseases, such as multiple sclerosis, Alzheimer's disease, Parkinson's disease, amyotrophic lateral sclerosis, dementia, cortico-basal degeneration, Huntington's disease, epilepsy; pathological conditions can also include psychiatric diseases, such as schizophrenia; peripheral inflammatory processes, such as pulmonary inflammation, atherosclerosis, cardiac ischemia, renal ischemia, rheumatism (fibromyalgia), psoriasic arthritis, rheumatoid arthritis and osteoarthritis; proliferative diseases, such as cancer.

-Receptor occupancy: a measure of the quantity of binding to a biochemical target or receptor. The level of receptor occupancy for a drug can be measured by comparing the time-activity measurements obtained with a radiolabelled PET ligand alone and when the PET ligand is given after administration of the unlabelled drug. The PET ligand must bind to the same receptor as the unlabelled drug. The amount of the radiolabelled PET ligand binding to the receptor decreases in the presence of increases concentrations of the unlabelled drug. The magnitude of this decrease is defined as the receptor occupancy of the unlabelled drug.

-PET imaging: positron emission tomography is an imaging technique which produces a three-dimensional image or map of functional processes in the body. The system detects pairs of gamma rays emitted indirectly by a positron-emitting radionuclide (tracer), which is introduced into the body on a biologically active molecule. Images of tracer concentration in 3-dimensional space within the body are then reconstructed by computer analysis. The materials used for PET are scintillators like bismuth germanate, lutetium oxy-orthosilicate, or gadolinium-orthosilicate, because of their high γ-ray stopping power and speed of signal conversion. Micro PET is the application of PET for small animal imaging.

-SPECT imaging: single photon emission computed tomography is another imaging technique which produces a three-dimensional image or map of functional processes in the body. The system directly detects gamma rays emitted by a single photon-emitting radionuclide (tracer), which is introduced into the body on a biologically active molecule. Images of tracer concentration in 3-dimensional space within the body are also then reconstructed by computer analysis.

-Radiolabelled form: any molecule where one or several atoms have been replaced by isotopes enabling its detection. The positron emitting radioisotopes most frequently used for PET imaging are: carbon-11 (or ¹¹C, t_{1/2}=20 min), nitrogen-13 (or ¹³N, t_{1/2}=10 min), oxygen-15 (or ¹⁵O, t_{1/2}=2 min). Are also to be considered molecules were additional atoms have been added in order to label the derivatives with other positron emitting radioisotopes for PET imaging, such as fluorine-18 (or ¹⁸F, t_{1/2}=110 min), but also gallium-68 (⁶⁸Ga, t_{1/2}=68 min), copper-64 (⁶⁴Cu, t_{1/2}=12.7 hrs), bromine-76 (or ⁷⁶Br, t_{1/2}=16.1 hrs) and iodine-124 (or ¹²⁴I, t_{1/2}=4.2 days). Finally, are also to be considered molecules were others additional atoms have been added in order to label the derivatives with single photon emitting radioisotopes for SPECT imaging, such as iodine-123 (or ¹²³I, t_{1/2}=13.1 hrs) or technetium-99m (or ^{99m}Tc, t_{1/2}=6.0 hrs).

The synthesis of radiolabelled ligands and the substitution of an atom by an isotope can be performed by several techniques known from skilled man in the art. For example, for the substitution of a carbon atom by a carbon-11, we can use several derivatives such as [¹¹C]methyl iodide or [¹¹C]methyl triflate (Welch M.J.et al. (2003 In Handbook of Radiopharmaceuticals - Radiochemistry and Applications (Welch MJ, Redvanly CS Eds.), New York-Chichester-Brisbane-Toronto, Wiley-Interscience Pub., 1-848). In the case of A, several methyl groups can be labelled with carbon-11, such as the N,N-dimethylacetamide or the N-methylindole functions.

In the case of a labelling with fluorine-18, the radioisotope may be directly attached to the core structure (A) by nucleophilic aliphatic or aromatic (including heteroaromatic (Dollé F. et al. (2005) Curr. Pharm. Design 11: 3221-3235)) substitutions or electrophilic substitutions or linked thought the addition of a spacer group, both techniques known from skilled man in the art (Kilbourn MR. (1990) In Fluorine-18 Labeling of Radiopharmaceuticals, Nuclear Science Series (Kilbourn MR Ed.), National Academy Press, Washington, D.C., 1-149; Lasne M.-C. et al. (2002) Topics in Current Chemistry, 222: 201-258; Cai L. et al. (2008) Eur. J. Org. Chem. 17: 2853-2873; Dollé F. et al. (2008) In Fluorine and Health: Molecular Imaging, Biomedical Materials and Pharmaceuticals, Tressaud A, Haufe G (Eds). Elsevier: Amsterdam-Boston-Heidelberg-London-New York-Oxford-Paris-San Diego-San Francisco-Singapore-Sydney-Tokyo, 3-65). Of particular interest is the use of an alkyl, alkenyl or alkyne linker for the addition of the fluorine-18 atom (Damont A. et al. (2008) J. Label. Compds Radiopharm.; 51: 286-292; Dollé F. et al. (2006) Bioorg. Med. Chem. 14:1115-1125; Dollé F. et al. (2007) J. Label. Compds Radiopharm. 50:716-723*).*

In the case of a labelling with another halogen (such as bromine-76, iodine-123 or iodine-124), the radioisotope may also be directly attached by nucleophilic or electrophilic substitutions to the core structure (A) or linked thought the addition of a spacer group, both techniques known from skilled man in the art (Mazière B. et al. (2001) Curr. Pharm. Des. 7: 1931-1943; Coenen H.H. et al. (2006) In Radioiodination reactions for pharmaceuticals - Compendium for effective synthesis strategies, Coenen H.H., Mertens J., Mazière B. (Eds), Springer Verlag, Berlin-Heidelberg, 1-101).

In the case of the labelling with metal radioisotopes (such as gallium-68, copper-64 or technetium-99m), the preferred approach used which will be considered by a skill man in the art, is the use of a bifunctional chelating agent based on for example the open-chain polyaminocarboxylates ethylenediamine tetraacetic acid (EDTA) and diethylenetriamine pentaacetic acid (DTPA), the polyaminocarboxylic macrocycle 1,4,7,10-tetraazacyclododecane-1,4,7,10-tetraacetic acid (DOTA), mercaptoacetyldi- and triglycine (MAG2, MAG3), bis-(S-benzoylthioglycoloyl)diaminopropanoate ((SBT)₂DAP) and hydrazinonicotinic acid (HYNIC), facilitating the complexation of the radiometal cation at one function and the covalent attachment to the core molecule at another (Brunner U.K. et al. (1995) Radiotracer production - Radiometals and their chelates In Principle of Nuclear Medecine, Wagner H.N. (Ed). Saunders: Philadelphia, 220-228); Weiner R.E. et al. (2003) Chemistry of gallium and indium radiopharmaceuticals In Handbook of Radiopharmaceuticals - Radiochemistry and Applications (Welch MJ, Redvanly CS Eds.), New York-Chichester-Brisbane-Toronto, Wiley-Interscience Pub., 363-400; Anderson C.J. et al. (2003) Chemistry of copper radionucleides and radiopharmaceuticals products In Handbook of Radiopharmaceuticals - Radiochemistry and Applications (Welch MJ, Redvanly CS Eds.), New York-Chichester-Brisbane-Toronto, Wiley-Interscience Pub., 401-422; Mahmood A. et al. (2003) Technetium radiopharmaceuticals In Handbook of Radiopharmaceuticals - Radiochemistry and Applications (Welch MJ, Redvanly CS Eds.), New York-Chichester-Brisbane-Toronto, Wiley-Interscience Pub., 323-362).

Direct addition of the fluorine-18 atom (or bromine-76, iodine-123 or iodine-124) may performed for example on the 3-phenyl aromatic ring or/and the pyridazino[4,5-b]indole aromatic rings as well as on any other chemically accessible position (such as the acetamide function). Indirect addition of these radiohalogens (for example through the use a spacer group) or addition of the metal radioactive isotopes mentioned above (gallium-68, copper-64 or technetium-99m, through the use of a chelating agent) may also be performed on any chemically accessible position of the N,N,5-trimethyl-4-oxo-3-phenyl-3,5-dihydro-4H-pyridazino[4,5-b]indole-1-acetamide (A) core (see references above).

-Administration: by preference the administration of a radiolabelled biomarker is by an intraveinous route of administration.

An first embodiment is the use of 7-chloro-N,N,5-trimethyl-4-oxo-3-phenyl-3,5-dihydro-4H-pyridazino[4,5-*b*]indole-1-acetamide as a biomarker for the detection, in an individual, of the PBR (peripheral benzodiazepine receptor) levels and inflammation associated with pathological conditions, wherein said compound is radiolabelled, preferentially radiolabelled with carbon-11 and more preferentially radiolabelled with carbon-11 on the carbon of the methyl group situated on position 5 of the indole nucleus.
In another embodiment, 7-chloro-N,N,5-trimethyl-4-oxo-3-phenyl-3,5-dihydro-4H-pyridazino[4,5-*b*]indole-1-acetamide is radiolabelled with radiohalogens, preferentially radiolabelled with the radiohalogen fluorine-18.
In some embodiments, the detection of PBR levels and inflammation is performed by PET imaging (positron emission tomography) or by SPECT imaging (single photon emission computed tomography).
In some embodiments of the invention, a radiolabelled form of 7-chloro-N,N,5-trimethyl-4-oxo-3-phenyl-3,5-dihydro-4H-pyridazino[4,5-b]indole-1-acetamide is used as a biomarker for the detection of PBR levels changes and inflammation associated with pathological conditions, wherein said pathological conditions are selected from brain injuries, such as stroke, ischemia-reperfusion injury, traumatic brain injury; brain infections, such as encephalitis; neurological diseases, such as multiple sclerosis, Alzheimer's disease, Parkinson's disease, amyotrophic lateral sclerosis, dementia, cortico-basal degeneration, Huntington's disease, epilepsy; psychiatric diseases, such as schizophrenia; peripheral inflammatory processes such as pulmonary inflammation, atherosclerosis, cardiac ischemia, renal ischemia, rheumatism (fibromyalgia), psoriasic arthritis, rheumatoid arthritis and osteoarthritis; proliferative diseases such as cancer.
In some embodiments of the invention a radiolabelled form of 7-chloro-N,N,5-trimethyl-4-oxo-3-phenyl-3,5-dihydro-4H-pyridazino[4,5-b]indole-1-acetamide is used as a biomarker of PBR levels and inflammation, wherein inflammation is neuroinflammation.
In some embodiments of the invention a radiolabelled form of 7-chloro-N,N,5-trimethyl-4-oxo-3-phenyl-3,5-dihydro-4H-pyridazino[4,5-b]indole-1-acetamide is performed for evaluating the efficacy of a therapeutic treatment

The present invention also concerns a method for the detection of the PBR and inflammation associated pathological conditions using a radiolabelled form of 7-chloro-N,N,5-trimethyl-4-oxo-3-phenyl-3,5-dihydro-4H-pyridazino[4,5-b]indole-1-acetamide.

In some embodiments, the radiolabelled form of 7-chloro-N,N,5-trimethyl-4-oxo-3-phenyl-3,5-dihydro-4H-pyridazino[4,5-b]indole-1-acetamide contains carbon-11.
In some embodiments, the radiolabelled form of 7-chloro-N,N,5-trimethyl-4-oxo-3-phenyl-3,5-dihydro-4H-pyridazino[4,5-b]indole-1-acetamide is radiolabelled on the carbon of the methyl group situated on position 5 of the indole nucleus.
In some embodiments of the invention, the pathological conditions are selected from brain injuries, such as stroke, ischemia-reperfusion injury, traumatic brain injury; brain infections, such as encephalitis; neurological diseases, such as multiple sclerosis, Alzheimer's disease, Parkinson's disease, amyotrophic lateral sclerosis, dementia, cortico-basal degeneration, Huntington's disease, epilepsy; psychiatric diseases, such as schizophrenia; peripheral inflammatory processes such as pulmonary inflammation, atherosclerosis, cardiac ischemia, renal ischemia, rheumatism (fibromyalgia), psoriasic arthritis, rheumatoid arthritis and osteoarthritis; proliferative diseases such as cancer.
Another embodiment according to the invention is a method of detection of the PBR and inflammation associated with pathological conditions, wherein inflammation is neuroinflammation.
Another embodiment according to the invention is a method of detection of the PBR and inflammation associated with pathological conditions, which is performed for occupancy studies.
Another embodiment is a method of detection of the PBR and inflammation associated with pathological conditions, said method comprising the following steps:
a) Administrating a radiolabelled form of 7-chloro-N,N,5-trimethyl-4-oxo-3-phenyl-3,5-dihydro-4H-pyridazino[4,5-*b*]indole-1-acetamide;
b) Acquiring of images in a region of interest in the brain or other peripheral tissues using the PET (or SPECT) technique;
c) Quantification of the levels of the PBR in a region of interest by quantifying the PET signal associated with a radiolabelled form of 7-chloro-N,N,5-trimethyl-4-oxo-3-phenyl-3,5-dihydro-4H-pyridazino[4,5-b]indole-1-acetamide in this region of interest;
d) Comparing the PET (SPECT) signal obtained in step c) with the signal obtained in a control region of interest ;
e) Determining the presence of inflammation associated with pathological conditions.

The present invention also relates to a diagnostic kit for the detection of PBR levels associated with normal conditions and changes in PBR levels associated with pathological conditions comprising a radiolabelled form of 7-chloro-N,N,5-trimethyl-4-oxo-3-phenyl-3,5-dihydro-4H-pyridazino[4,5-b]indole-1-acetamide.

The following examples further illustrate the present invention and are not intended to limit the invention. For convenient reasons and to facilitate reading, the compound 7-chloro-N,N,5-trimethyl-4-oxo-3-phenyl-3,5-dihydro-4H-pyridazino[4,5-b]indole-1-acetamide has been renamed as "A" into the figures and the results.

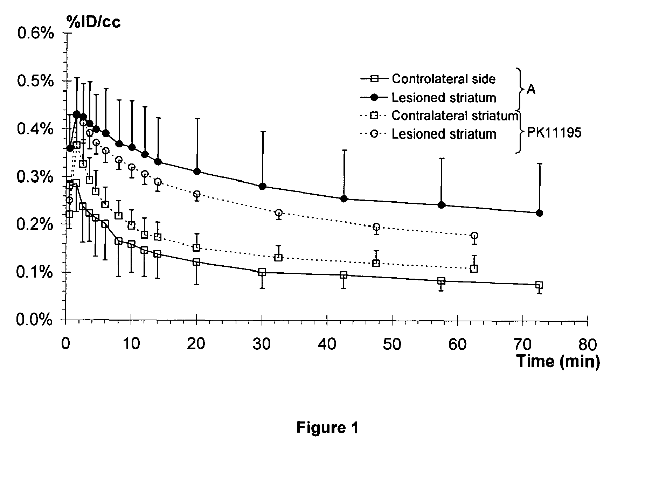

**Figure 1****:** Time-activity-curves of [¹¹C]-A and [¹¹C]PK11195 in lesioned and intact rat striatum. Data are expressed as percentage of injected dose per cubic centimeter as a function of post-injection time (min).

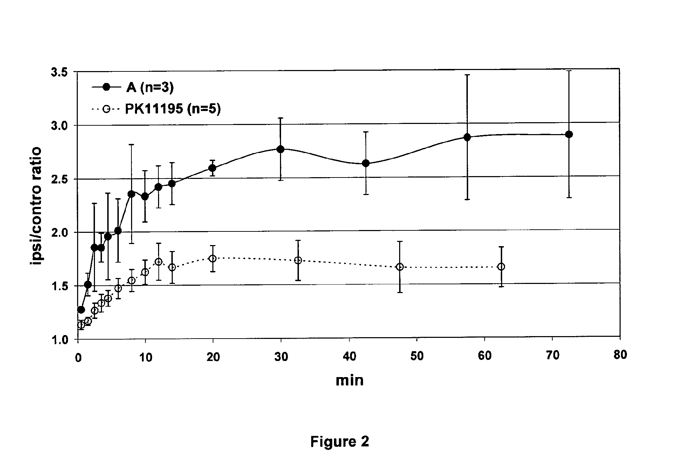

**Figure 2****:** The ratio of [¹¹C]-A and [¹¹C]PK11195 uptake in lesioned vs intact controlateral rat striatum.

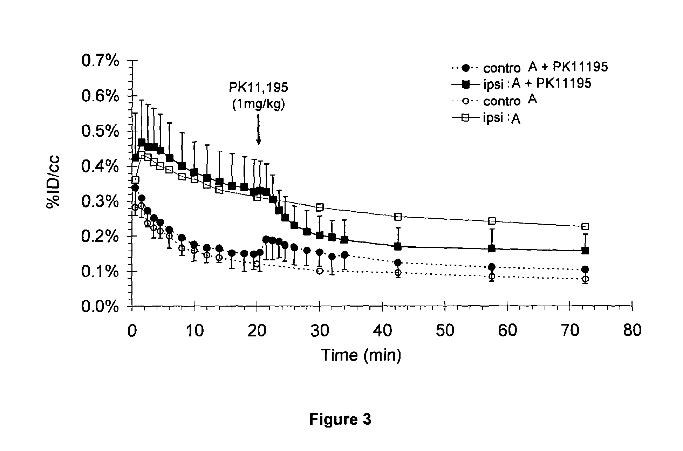

**Figure 3****:** Time-activity-curves of [¹¹C]-A in lesioned (ipsi) and intact rat striatum (contro). Arrow indicates addition of an excess of 1mg/kg of PK11195 20 min after injection of [¹¹C]-A in lesioned (ipsi + PK) and intact contralateral (contro + PK) rat striatum. Data are expressed as percentage of injected dose per cubic centimeter as a function of post-injection time (min).

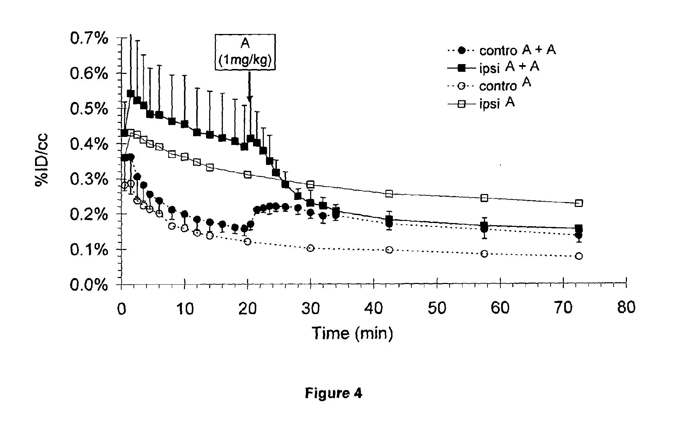

**Figure 4****:** Time-activity-curves of [¹¹C]-A in lesioned (ipsi SSR) and intact rat striatum (contro SSR). Arrow indicates addition of an excess of 1mg/kg of A 20 min after injection of [¹¹C]-A in lesioned (ipsi A + A) and intact contralateral (contro A+ A) rat striatum. Data are expressed as percentage of injected dose per cubic centimeter as a function of post-injection time (min)

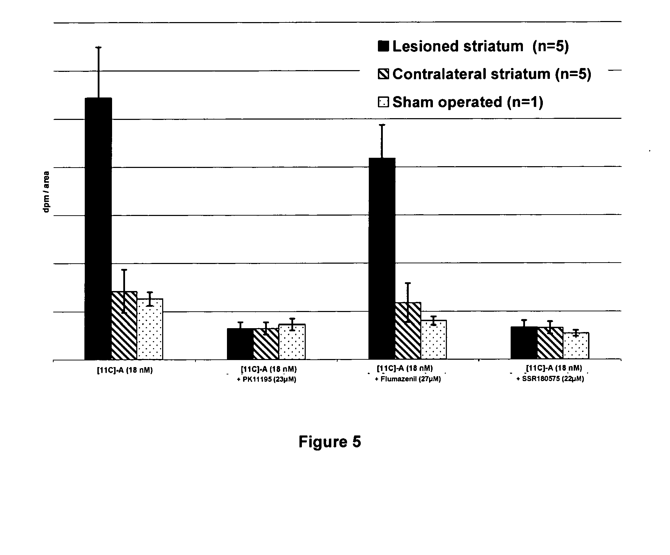

**Figure 5****:** [¹¹C]-A (18 nM) autoradiography in rat brain sections (20 µm) 7-8 days post-lesion. Non-specific binding was assessed using an excess of either unlabelled PK11195 (23 µM) or A (22 µM). Specificity for PBR vs central benzodiazepine binding sites was evaluated using an excess of unlabelled Flumazenil (27 µM). Data are expressed as dpm per arbritary area unit. * indicates a significant difference relative to the [¹¹C]-A (18 nM) binding in the lesioned striatum.

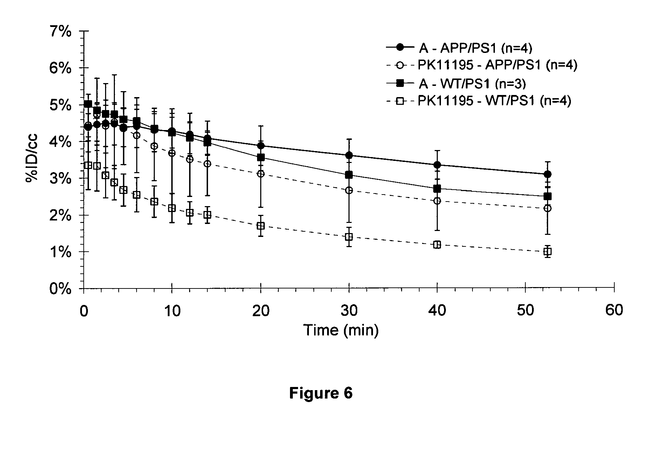

**Figure 6****:** Time-activity-curves of [¹¹C]-A and [¹¹C]PK11195 in the whole brain (cerebellum excluded) of 11-12 month APP/S1 and wild type PS1 transgenic mice. Data are expressed as percentage of injected dose per cubic centimeter as a function of post-injection time (min).

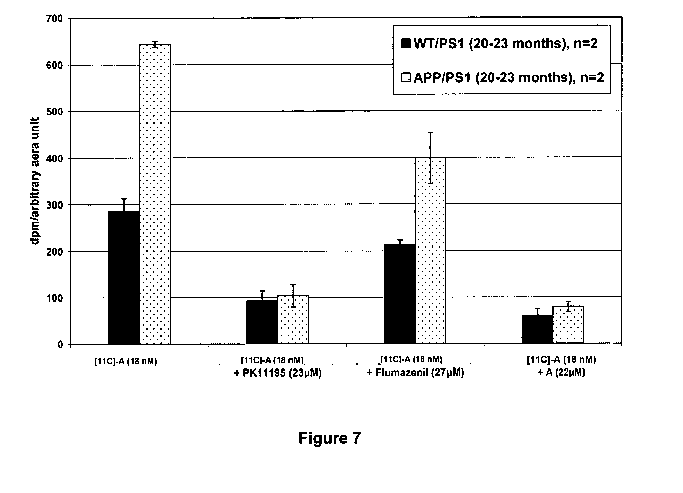

**Figure 7****:** [¹¹C]-A 18 nM) autoradiography in whole brain sections from 20-23 month old APP/PS1 and wild type PS1 transgenic mice. Non-specific binding was assessed using an excess of either unlabelled PK11195 (23 µM) or A (22 µM). Specificity for PBR vs central benzodiazepine binding sites was evaluated using an excess of unlabelled Flumazenil (27 µM). Data are expressed as dpm per arbritary area unit.

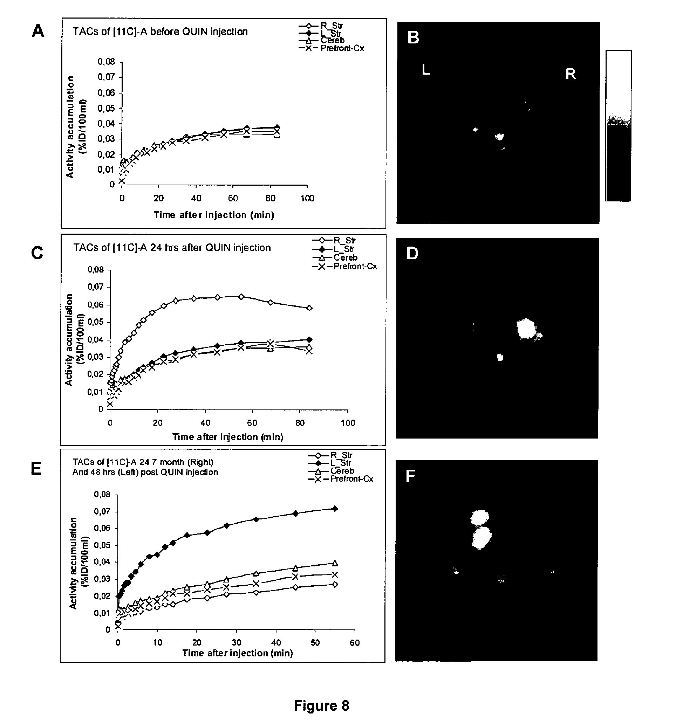

**Figure 8****:** Time-activity-curves of [¹¹C]-A in 4 different brain regions of a non-human primate (right and left striatum, prefrontal cortex, cerebellum) and corresponding PET summation images of selected coronal brain slices over 120 min at different time-points of the study : baseline (4A,B) and 24 hrs post lesion in the right striatum (4C,D). The panel (4E,F) displays the [¹¹C]-A time activity curves at 48 hrs post lesion in the left striatum and at 7 months post lesion in the right striatum. Data are expressed as percentage of injected dose per 100ml (%ID/100ml) as a function of post-injection time (min).

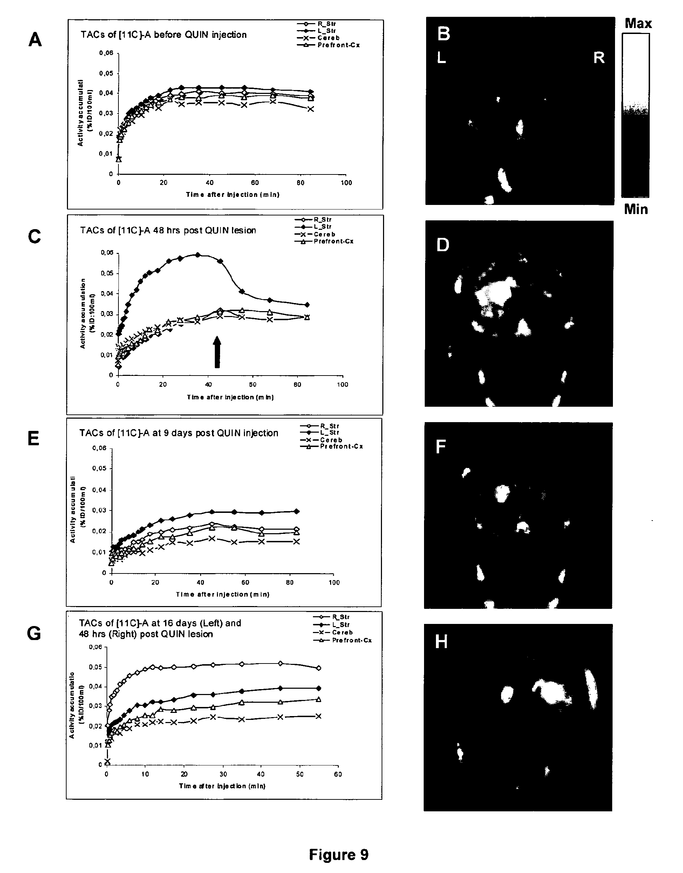

**Figure 9****:** Time-activity-curves of [¹¹C]-A in 4 different brain regions (right and left striatum, prefrontal cortex, cerebellum) and corresponding PET summation images of selected coronal brain slices over 120 min at four different time-points of the study : baseline (4A,B), at 48 hrs post lesion (4C,D), at 9 days post lesion (4E,F) and at 16 days post lesion in the left striatum and at 48 hrs post lesion in the right striatum. Arrow indicates time of administration of excess unlabelled PK11195 (1 mg/kg). Data are expressed as percentage of injected dose per 100ml (%ID/100ml) as a function of post-injection time (min).

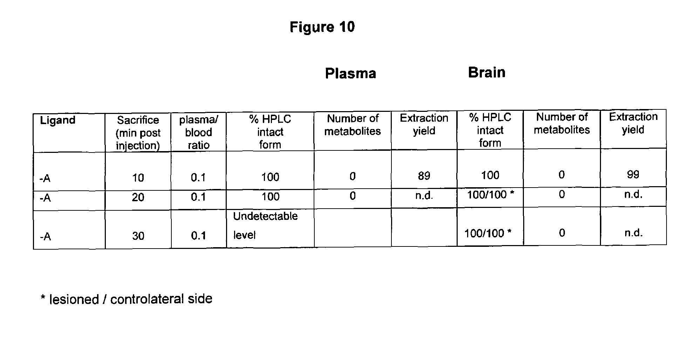

**Figure 10****:** Analysis of [¹¹C]-A metabolites in plasma and brain.

### Methods

### Radiosynthesis of ligands

### Labelling with carbon-11

**[¹¹C]PK11195 ((R)-*N*-[¹¹C]Methyl-*N*-(1-methylpropyl)-1-(2-chlorophenyl)isoquinoline-3-carboxamide, R-enantiomer).** Preparation of [¹¹C]PK11195 is based on minor modifications of published processes (Camsonne C. et al. (1984) J. Label. Comp. Radiopharm 21: 985-991; Cremer J.E. et al. (1992) Int. J. Rad. Appl. Instrum. B. 19: 159-66; Boutin, H. et al. (2007) Glia 55: 1459-68; Boutin, H. et al. (2007) J. Nucl. Med. 48: 573-581) and includes the following steps : (1) trapping at -10°C of [¹¹C]methyl iodide in DMF/DMSO (2/1 (v:v), 300 µl) containing 1.5 to 2.0 mg of the precursor for labelling and 15-20 mg of powdered hydroxide (excess) ; (2) heating at 110°C for 3 min ; (3) taking-up the mixture with 0.5 mL of the HPLC mobile phase and (4) purification using semi-preparative HPLC. Quality controls, in particular radiochemical and chemical purity determinations, were performed on an aliquot of the final production batch.
**[¹¹C]-A labelling at the *N*-methylindole function: 7-Chloro-*N,N*-dimethyl-5-[¹¹C]methyl-4-oxo-3-phenyl-3,5-dihydro-4H-pyridazino[4,5-*b*]indole-1-acetamide).** Preparation of [¹¹C]-A includes the following steps : (1) trapping at -10°C of [¹¹C]methyl triflate in DMF (300 µl) containing 0.2 to 0.3 mg of the precursor for labelling and 4 mg of powdered potassium carbonate (excess) ; (2) heating at 120°C for 3 min ; (3) taking-up the mixture with 0.5 mL of the HPLC mobile phase and (4) purification using semi-preparative HPLC. Quality controls, in particular radiochemical and chemical purity determinations, were performed on an aliquot of the final production batch.
**[¹¹C]-A (Labelling at the *N,N*-dimethylacetamide function: 7-Chloro-*N*-[¹¹C]methyl-*N*,5-dimethyl-4-oxo-3-phenyl-3,5-dihydro-4H-pyridazino[4,5-*b*]indole-1-acetamide).**
Preparation of [¹¹C]-A also includes the following steps : (1) trapping at -10°C of [¹¹C]methyl iodide in a 1/2 (v:v) mixture of DMF and DMSO (100/200 µl) containing 0.5 to 1.0 mg of the precursor for labelling and 5 µL of an 1M tetrabutylammoniumhydroxide solution in methanol ; (2) heating at 120°C for 3 min ; (3) taking-up the mixture with 0.5 mL of the HPLC mobile phase and (4) purification using semi-preparative HPLC. Quality controls, in particular radiochemical and chemical purity determinations, were performed on an aliquot of the final production batch.

### Labelling with fluorine-18

Preparation of all fluorine-18-labelled derivatives of A includes at least the following steps : (1) fluorination using a [¹⁸F]fluoride source at moderate to high temperature in a selected solvent (300 to 900 µl) containing 1 to 10 mg of the appropriate precursor for labelling and (2) purification using for example semi-preparative HPLC. As described above, quality controls, in particular radiochemical and chemical purity determinations, were performed on an aliquot of the final production batch.
There is no particular restriction on the nature of the sources of [¹⁸F]fluoride anions to be used in this reaction, and any sources of [¹⁸F]fluoride anions conventionally used in reactions of this type may equally be used here, provided that it has no adverse effect on other parts of the molecule. Examples of suitable sources of [¹⁸F]fluoride anions include: alkali metal [¹⁸F]fluorides, such as sodium [¹⁸F]fluoride, potassium [¹⁸F]fluoride, cesium [¹⁸F]fluoride; ammonium [¹⁸F]fluoride, tetraalkylammonium [¹⁸F]fluorides, such as tetrabutylammonium [¹⁸F]fluoride. Of these, the alkali metal [¹⁸F]fluorides, and notably a potassium fluoride, are preferred. The source of [¹⁸F]fluoride anions may be activated by the presence of a ligand able to complex the counter cationic species of the source of [¹⁸F]fluoride anions. The ligand may be notably a cyclic or polycyclic multidentate ligand. Examples of suitable ligands include notably crown ethers such as 1,4,7,10,13-pentaoxacyclooctadecane (18-C-6) or cryptands such as 4,7,13,16, 21,24-hexaoxa-1,10-diazabicyclo-[8,8,8]hexacosane sold under the name K222^{®}. Preferably, the source of [¹⁸F]fluoride anions is an alkaline metal [¹⁸F]fluoride-cryptate complex, notably a potassium [¹⁸F]fluoride-cryptate complex, preferably the potassium [¹⁸F]fluoride - 4,7,13,16,21,24-hexaoxa-1,10-diazabicyclo-[8,8,8]hexacosane (K[¹⁸F]/K222). The complex K[¹⁸F]/K222^{®} may be prepared by any conventional methods (Dollé F. et al. (1999) J. Med. Chem. 42: 2251-2259 or Dolci L. et al. (1999) Bioorg. Med. Chem. 7: 467-479).
The fluorination reaction can be performed in various solvents and can take place over a wide range of temperatures. In general, it is convenient to carry out the reaction at a temperature of from about 50°C to about 200°C and the more often used solvents are dimethylsulfoxide (DMSO), dimethylformamide (DMF) and acetonitrile. The time required for the reaction may also vary widely (from about 5 min to 15 min for example), depending on many factors, notably the reaction temperature, the nature of the reagents and solvents and the amount of the labelling precursor used (Kilbourn MR. (1990) In Fluorine-18 Labeling of Radiopharmaceuticals, Nuclear Science Series (Kilbourn MR Ed.), National Academy Press, Washington, D.C., 1-149; Lasne M.-C. et al. (2002) Topics in Current Chemistry, 222: 201-258; Dollé F. et al. (2005) Curr. Pharm. Design 11: 3221-3235; Cai L. et al. (2008) Eur. J. Org. Chem. 17: 2853-2873; Dollé F. et al. (2008) In Fluorine and Health: Molecular Imaging, Biomedical Materials and Pharmaceuticals, Tressaud A, Haufe G (Eds). Elsevier: Amsterdam-Boston-Heidelberg-London-New York-Oxford-Paris-San Diego-San Francisco-Singapore-Sydney-Tokyo, 3-65). The radiofluorinated compounds thus prepared are usually purified by HPLC as decribed for the carbone-11-labelled derivatives, but may also be recovered or pre-purified from the reaction mixture by the use of other known chromatography techniques or simply by filtration on a pre-packed separation column.
**[¹⁸F]Fluoroethoxy-A (7-chloro-*N,N*,5-trimethyl-4-oxo-3-(4-[¹⁸F]fluoroethoxy)-phenyl-3,5-dihydro-4H-pyridazino[4,5-b]indole-1-acetamide)**
Preparation of [¹⁸F]fluoroethoxy-A includes the following steps : (1) taking up the K[¹⁸F]F-Kryptofix^{®}222 complex with a DMSO solution (600 µL) containing the tosyloxy precursor for labelling (2.0-8.0 mg) ; (2) heating at 165°C for 3-10 min ; (3) pre-purification using a C-8 or C-18 PrepSep cartridge and (4) purification using semi-preparative HPLC. Quality controls, in particular radiochemical and chemical purity determinations, were performed on an aliquot of the final production batch.

### Labelling with other halogens (bromine-76, iodine-123, iodine-124)

Preparation of all other radiohalogenated derivatives (bromine-76, iodine-123, iodine-124) followed standards techniques and procedures known skilled man in the art (Mazière B. et al. (2001) Curr. Pharm. Des. 7: 1931-1943; Coenen H.H. et al. (2006) In Radioiodination reactions for pharmaceuticals - Compendium for effective synthesis strategies, Coenen H. H., Mertens J., Mazière B. (Eds), Springer Verlag, Berlin-Heidelberg, 1-101).

### Labelling with radiometals (gallium-68, copper-64 and technetium-99m)

Preparation of derivatives labelled with radiometals (gallium-68, copper-64 and technetium-99m) also followed standards techniques and procedures known skilled man in the art (Brunner U.K. et al. (1995) Radiotracer production - Radiometals and their chelates In Principle of Nuclear Medecine, Wagner H.N. (Ed). Saunders: Philadelphia, 220-228); Weiner R.E. et al. (2003) Chemistry of gallium and indium radiopharmaceuticals In Handbook of Radiopharmaceuticals - Radiochemistry and Applications (Welch MJ, Redvanly CS Eds.), New York-Chichester-Brisbane-Toronto, Wiley-Interscience Pub., 363-400; Anderson C.J. et al. (2003) Chemistry of copper radionucleides and radiopharmaceuticals products In Handbook of Radiopharmaceuticals - Radiochemistry and Applications (Welch MJ, Redvanly CS Eds.), New York-Chichester-Brisbane-Toronto, Wiley-Interscience Pub., 401-422; Mahmood A. et al. (2003) Technetium radiopharmaceuticals In Handbook of Radiopharmaceuticals - Radiochemistry and Applications (Welch MJ, Redvanly CS Eds.), New York-Chichester-Brisbane-Toronto, Wiley-Interscience Pub., 323-362).

### Formulation

Formulation of [¹¹C]PK11195, [¹¹C]-A or any other radiolabelled A derivatives as an i.v. solution for injection often includes a Waters SepPak^{®} cartridge-based removal of the HPLC solvents and/or a simple dilution with aq. 0.9% NaCl (physiological saline) to an ethanol concentration below 10%.

### Animal Models

All studies were conducted in accordance with the French legislation and European directives.

### Rat model of neuroinflammation

Wistar rats (average body weight 300g, centre d'Élevage René Janvier, France) were kept in thermoregulated, humidity controlled facilities under a 12h/12h light/dark cycle (light on between 7h AM and 7h PM) and were allowed free access to food and water.

Neuroinflammation was induced by stereotaxic injection of AMPA (alpha-amino-3-hydroxy-5-methyl-4-isoxazolepropionate) (15mM in PBS buffer, Sigma®) using a 1µl microsyringe and micropump (injection rate: 0.5µl/min, UltraMicroPump II® and Micro4® Controller, WPI Inc., USA), as previously described (Boutin, H. et a/. (2007) Glia 55: 1459-68.; Boutin, H. et al. (2007) J. Nucl. Med. 48: 573-581). AMPA (0.5µl) was injected into the right striatum (Bregma +0.7mm, from sagittal suture: 2.7mm, depth from brain surface: 5.5mm). Animals were maintained normothermic (body temperature: 36.7±0.5°C, mean±SD) during the surgery through the use of a heating blanket (Homeothermic Blanket Control Unit, Harvard Apparatus Limited@, Edenbridge, Kent, UK).

### Primate model of neuroinflammation

Cynomolgus macaques *(Macaca fascicularis)* weighing 4-5 kg were housed in thermoregulated, humidity controlled facilities under a 12 h/12 h light/dark cycle (light on between 7 h a.m. and 7 h p.m.) and were allowed free access to food and water. Neuroinflammation was induced by local sterotaxic injections of quinolinic acid (quinolinic acid, Sigma, St. Louis, MO; dissolved in 0.1 M PBS, pH 7.2) into the primate striatum (1 injection site into caudate and 2 injection sites into the putamen) during two different surgical interventions where quinolinic acid was injected into one hemisphere on day 1 and the second hemisphere 2 weeks or 7 months later (Fig. 8 and 9). During each surgical session the animals received 60 nmol of quinolinic acid distributed over three different striatal sites, one 5µl site in the caudate and two 10 µl sites in the putamen using a 10 µl-Hamilton syringe attached to a 26-gauge needle. The stereotactic coordinates, determined according to the stereotactic atlas of Swabo and Cowan (1984), were as follows: caudate site [AP +19 mm, ML ±6 mm, DV -14 mm from sinus] ; putaminal site1 [AP: +19 mm, ML: ±12 mm, DV: -17 mm from stereotaxic zero] ; putaminal site 2 [AP: +17 mm, ML: ±13 mm, DV: -16 mm from sinus]. The excitotoxin was injected at a rate of 0.1 µl/min and the injection syringe left in place for an additional 5 min to avoid backflow of the toxin. Throughout the surgery, the primate temperature was maintained normothermic (rectal temperature, 36°C ± 0.6°C, mean ± SD). After removing the needle, the skin was sutured and the animals were allowed to recover from anesthesia and returned to their cages when fully awake.

### Transgenic mice

Generation and characterization of single PS1M146L (PS1) and double APP751SLxPS1M146L (APPxPS1) transgenic mice were, as described previously (Blanchard, V. et al. (2003) Exp. Neurol. 184: 247-263). In these animals APP is expressed at a high level in all cortical neurons under the control of the Thy-1 promoter. Human PS1 with the M146L mutation is expressed under the control of the HMG-CoA reductase promoter. The level of amyloid load was found to be quite reproducible at a given age. Both single PS1 and double APPxPS1 mice, provided by Sanofi-Aventis, were used for PET imaging at the age of 11-12 months and autoradiography at 20-23 months.

### MicroPET scans and data acquisition

### Rats and transgenic mice

MicroPET imaging was performed 7 days post-AMPA injection in rats, and in 11-12 month old transgenic mice. In both mice and rats, anaesthesia was induced by isoflurane 5%, and thereafter maintained by 2-2.5% of isoflurane in a mixture 70%/30% NO₂/O₂. For PET scans, the head of the rat was placed in a home-made stereotaxic frame compatible with PET acquisition and the rats were maintained normothermic (rectal temperature: 36.7±0.5°C, mean±SD). Mice were placed on a bed equipped with an anaesthetic mask allowing heating of the air flow and the rectal temperature was monitored using the Homeothermic Blanket Control Unit. All imaging protocols were conducted with a Concorde Focus 220 PET scanner using either [¹¹C]PK11195 or [¹¹C]-A.
In rats, radiolabelled compounds and unlabelled ligands were injected in the caudal vein with the use of 24 gauge catheter. Radiolabelled compounds were injected concomitantly at the start of the PET acquisition and unlabelled compounds were injected 20min after injection of radiotracers. PET data were acquired for 80min. In mice, radiolabelled compounds were injected in the caudal vein using 28 gauge needles immediately before the PET scan initiation. PET data were acquired for 60min.

### Primates

Imaging sessions were performed before and at various times after quinolinic acid injection (24 hrs, 48 hrs, 9 days, 16 days and 7 months post lesion). [¹¹C]-A was injected and brain kinetics followed by PET for 90 min.
One hour before PET imaging, animals were anaesthetized by intramuscular injection of a ketamine / xylazine mixture (15mg/kg / 1.5mg/kg) and intubated. Catheters were then placed in a saphenous vein for radiotracer injection and in a femoral artery for blood sampling. Animals were maintained anesthetized by an intravenous injection of propofol (Diprivan® 1%; 0.05 mg/kg.min-1).
To ensure correct positioning of the animal in the apparatus, the animal's head was secured in a home-made stereotactic frame. PET scans were performed using the high-resolution Focus micro-PET (CTI-Siemens, Knoxville, TN), which acquires 95 contiguous planes simultaneously. For attenuation correction, a transmission scan was first performed using a ⁶⁸Ge rotating rod source. Macaques were intravenously injected with 192.29 ± 33.67 MBq of [¹¹C]-A and the acquisition performed for 90 min. All PET acquisitions were performed in list mode (3D mode) and images were reconstructed using the following time frame: (4 images of 25 s) + (4 images of 30 s) + (2 images of 1 min) + (5 images of 2 min) + (3 images of 5 min) + (3 images of 10 min) and (1 image of 15 min), for a total time of 90 min for [¹¹C]-A.

### Image analysis

PET image analysis was performed using ASIPro VM™ (CTI Concorde Microsystems' Analysis Tools and System Setup/Diagnostics Tool) and Brainvisa/Anatomist (http://brainvisa.info/).

### Metabolite analysis in rat blood and brain

Naive or operated adult male Wistar rats (body weight 300-400g) were injected i.v in the tail vein with [¹¹C]-A. Animals were sacrificed 10, 20 or 30 min later. A blood sample was collected and plasma isolated by centrifugation (5min, 3000rpm). Plasma proteins were precipitated from 400µl of serum by addition of 400µl of acetonitrile. After centrifugation (5min, 3000rpm), the supernatant was injected onto the HPLC column. Rat brains were removed and hemispheres were separated. Homogenisation by sonication was performed in 1 ml of acetonitrile per hemisphere. After a rapid centrifugation, the supernatant was separated from the pellet and concentrated under reduced pressure before injection onto the HPLC (see radiochemistry section for HPLC conditions).

### Autoradiography

[¹¹C]-A autoradiography was performed using 20 µm brain sections from rats (7-8 days post-lesion) or mice (20-23 months). Non-specific binding was assessed using an excess of either unlabelled PK11195 or A. Specificity for PBR vs central benzodiazepine binding sites was evaluated by using an excess of unlabelled Flumazenil. Sections were incubated for 20 min in Tris Buffer (TRIZMA pre-set Crystals, Sigma®, adjusted at pH7.4 at 4°C, 50 mM with NaCl 120 mM), then rinsed 2 times for 2 min with cold buffer, followed by a quick wash in cold distilled water. Sections were then placed in direct contact with a Phosphor-Imager screen and exposed overnight; autoradiograms were analysed using ImageQuant™ software.software.

### EXAMPLE 1

[¹¹C]PK11195 radiosynthesis: Final HPLC purification of [¹¹C]PK11195 was performed on a semi-preparative Waters Symmetry^{®} C-18 HPLC colum (eluant : water / acetonitrile / TFA : 40 / 60 / 0.1 [v:v:v] ; flow-rate : 7 ml/min) and the peak corresponding to radiochemically pure [¹¹C]PK11195 (Rt : 6.5-7.0 min) was collected. Typically, starting from a 55.5 GBq [¹¹C]CO₂ cyclotron production batch, about 4.5-5.0 GBq of [¹¹C]PK11195 were obtained within 30 min of radiosynthesis (including HPLC purification and formulation). Radiochemical purity (determined by analytical HPLC on Waters Symmetry-M^{®} C-18 column) was greater than 95% and specific radioactivities ranged from 50 to 90 GBq/µmol (at the end of the radiosynthesis).

### EXAMPLE 2

[¹¹C]-A radiosynthesis (Labelling at the N-methylindole function): Final HPLC purification of [¹¹C]-A was performed on a semi-preparative Zorbax^{®} SB-C-18 HPLC colum (eluant : 0.9% aq. NaCl / EtOH / 1M aq. phosphate buffer (pH 2,3) : 50 / 50 / 0.1 [v:v:v] ; flow-rate : 6 mL/min) and the peak corresponding to radiochemically pure [¹¹C]-A (Rt : 8.0-8.5 min) was collected. Typically, starting from a 55.5 GBq [¹¹C]CO₂ cyclotron production batch, about 4.5-6.0 GBq of [¹¹C]-A were obtained within 25 min of radiosynthesis (including HPLC purification and formulation). Radiochemical purity (determined by analytical HPLC on Waters Symmetry-M^{®} C-18 column) was greater than 95% and specific radioactivities ranged from 50 to 90 GBq/µmol (at the end of the radiosynthesis).

### EXAMPLE 3

[¹¹C]-A radiosynthesis (Labelling at the *N,N*-dimethylacetamide function): Final HPLC purification of [¹¹C]-A was performed on a semi-preparative SymmetryPrep^{®} C-18 HPLC colum (eluant : water / acetonitrile / TFA : 50 / 50 / 0.1 [v:v:v] ; flow-rate : 5 ml/min) and the peak corresponding to radiochemically pure [¹¹C]-A (Rt : 8.0-8.5 min) was collected. Typically, starting from a 55.5 GBq [¹¹C]CO₂ cyclotron production batch, about 3.5-5.0 GBq of [¹¹C]-A were obtained within 25 min of radiosynthesis (including HPLC purification and formulation). Radiochemical purity (determined by analytical HPLC on Waters Symmetry-M^{®} C-18 column) was greater than 95% and specific radioactivities ranged from 50 to 90 GBq/µmol (at the end of the radiosynthesis).

### EXAMPLE 4

**Fluoroethoxy-A synthesis.** To a suspension of K₂CO₃ (101 mg, 0.73 mmol) in dry DMF (8 mL) is added the 4-hydroxyderivative of A (150 mg, 0.36 mmol, see WO00/44384), in solution in dry DMF (2 mL). The reaction mixture is stirred for 30 min at room temperature and 2-fluoroethyl-4-methylbenzenesulfonate (96 mg, 0.44 mmol, synthesized according to Damont et al. - J. label Compds Radiopharm 2008, 51, 286-292) in solution in DMF (2 mL) is added. The reaction mixture is warmed to 60°C for 2 hrs and stirred an additional hour at room temperature. The mixture is then partitioned between 1M aq. HCl and CH₂Cl₂ and the organic phase is washed with saturated brine, dried over magnesium sulfate, filtered and evaporated to dryness. The residue is purified by silica gel column chromatography (CH₂Cl₂/MeOH 98:2 to 95:5 v/v as eluent) to afford the desired compound (105 mg, 63%) as a white powder. *R*_{f} : 0.38 (SiO₂-TLC (CH₂Cl₂/MeOH: 95/5 v:v)). ¹H NMR (CD₂Cl₂, 400 MHz) δ 7.89 (d, 1H, J: 8.8 Hz), 7.58 (d, 1H, J: 1.6 Hz), 7.54 (d, 2H, J: 9.2 Hz), 7.34 (dd, 1H, J: 8.8, 1.6 Hz), 7.03 (d, 2H, J: 9.2 Hz), 7.78 (dt, 2H, J²_{H-F}: 47.6, J_{H-H} : 4.0 Hz), 4.32 (s, 3H), 4.26 (dt, 2H, J³_{H-F}. 28.4, J_{H-H}: 4.0 Hz), 4.16 (s, 2H), 3.19 (s, 3H), 2.96 (s, 3H). ¹³H NMR (CD₂Cl₂, 400 MHz) δ 168.2 [C], 157.6 [C], 155.1 [C], 141.3 [C], 140.7 [C], 140.3 [C], 135.4 [C], 132.8 [C], 127.4 [2*CH], 123.2 [CH], 122.6 [CH], 118.9 [C], 117.2 [C], 114.3 [2*CH], 110.7 [CH], 82.0 [d, J¹_{C-F}: 169 Hz, CH₂F], 67.5 [d, J²_{C-F}: 20 Hz, CH₂CH₂F], 39.5 [CH₂], 37.4 [CH₃], 35.2 [CH₃], 31.6 [CH₃]. Anal. Calcd for C₂₃H₂₂CIFN₄O₃.0.15 H₂O: C, 60.11, H, 4.89, N, 12.19, found : C, 60.00, H, 4.96, N, 12.18.

**Tosyloxyethoxy-A synthesis**. To a suspension of K₂CO₃ (101 mg, 0.73 mmol) in dry DMF (8 mL) is added the 4-hydroxyderivative of A (150 mg, 0.36 mmol, see WO00/44384) in solution in dry DMF (2 mL). The reaction mixture is stirred for 30 min at room temperature and ethylene di(p-toluenesulfonate) (677 mg, 1.83 mmol, commercially available) in solution in DMF (2 mL) is added. The reaction mixture is warmed to 60°C for 2 hrs and stirred an additional hour at room temperature. The mixture is then partitioned between 1 M aq. HCl and CH₂Cl₂ and the organic phase is washed with saturated brine, dried over magnesium sulfate, filtered and evaporated to dryness. The residue is purified by silica gel column chromatography (CH₂Cl₂/MeOH 98:2 to 95:5 v/v as eluent) to afford the desired compound (100 mg, 45%) as a white fluffy solid. *R*_{f} : 0.72 (SiO₂-TLC (CH₂Cl₂/MeOH: 95/5 v:v)). ¹H NMR (CD₂Cl₂, 400 MHz) δ 7.89 (d, 1H, J: 8.8 Hz), 7.84 (d, 2H, J: 8.4 Hz), 7.60 (d, 1H, J: 1.6 Hz), 7.53 (d, 2H, J: 8.8 Hz), 7.41 (d, 2H, J: 8.4 Hz), 7.36 (dd, 1H, J: 8.8, 1.6 Hz), 6.91 (d, 2H, J: 8.8 Hz), 4.40 (t, 2H, J: 4.4 Hz), 4.32 (s, 3H), 4.22 (t, 2H, J: 4.4 Hz), 4.17 (s, 2H), 3.21 (s, 3H), 2.98 (s, 3H), 2.48 (s, 3H). ¹³H NMR (CD₂Cl₂, 400 MHz) δ 168.1 [C], 157.2 [C], 155.0 [C], 145.2 [C], 141.2 [C], 140.3 [C], 135.5 [C], 132.8 [C], 132.7 [C], 131.3 [C], 129.9 [2*CH], 127.9 [2*CH], 127.4 [2*CH], 123.2 [CH], 122.6 [CH], 118.9 [C], 117.2 [C], 114.4 [2*CH], 110.7 [CH], 68.3 [CH₂], 65.8 [CH₂], 39.5 [CH₂], 37.4 [CH₃], 35.2 [CH₃], 31.6 [CH₃], 21.3 [CH₃].

### EXAMPLE 5

[¹⁸F]Fluoroethoxy-A radiosynthesis: Final HPLC purification of [¹⁸F]fluoroethoxy-A was performed on a semi-preparative Symmetry^{®} C-18 HPLC colum (eluant : water / acetonitrile / TFA : 60 / 40 / 0.1 [v:v:v] ; flow-rate : 5 ml/min) and the peak corresponding to radiochemically pure [¹⁸F]fluoroethoxy-A (Rt : 11.0-13.0 min) was collected. Starting from a 37 GBq [¹⁸F]fluoride cyclotron production batch, about 3.7 GBq of [¹⁸F]fluoroethoxy-A were obtained within 90 min of radiosynthesis including HPLC purification and formulation). Radiochemical purity (determined by analytical HPLC on Waters Symmetry-M^{®} C-18 column) was greater than 95% and specific radioactivities greater than 50 GBq/µmol (at the end of the radiosynthesis).

### EXAMPLE 6

The uptake of [¹¹C]-A uptake is significantly higher in the lesioned striatum of AMPA-injected rats (the region in which PBR expression is induced) compared to the intact controlateral striatum which is expected to express no or non-significant amounts of PBR (Figure 1). The uptake of [¹¹C]-A is also significantly higher than the uptake observed with reference PBR PET ligand, [¹¹C]PK11195, in the lesioned striatum (Figure 1). The ratio of the uptake in the lesioned over the intact striatum is also significantly higher for [¹¹C]-A than for [¹¹C]PK11195 (Figure 2). The binding potential and R1 are significantly higher for [¹¹C]-A than for [¹¹C]PK11195 (BP = 1.65 ± 0.36 vs 0.66 ± 0.15; R1 = 1.26 ± 0.08 vs 1.10 ± 0.05).
Both unlabelled PK11195 (Figure 3) and A (Figure 4) (1mg/kg i.v.; 20 min post [¹¹C]-A injection) significantly reduced the brain uptake of [¹¹C]-A in the lesioned striatum. Both unlabelled compounds induced a small increase in [¹¹C]-A binding in the controlateral side which is probably due to an increase in the blood concentration of [¹¹C]-A due to the release of [¹¹C]-A from extra-cerebral binding sites.
Analysis of the metabolites present in the blood and the plasma of rats at 10 and 20 min after [¹¹C]-A injection and of the metabolites present in the brain of rats at 10, 20 and 30 min after [¹¹C]-A injection essentially detected the parent compound only (Figure 10).
In addition, autoradiography of [¹¹C]-A binding on brain sections mirrored the imaging results with a high ipsilateral to contralateral ratio (3.8), which is abolished by an excess of unlabelled PK11195 or A (Figure 5). A small, but significant reduction of [¹¹C]-A binding was observed with unlabelled flumazenil, a benzodiazepine antagonist (Figure 5)

### EXAMPLE 7

In both APPxPS1 and wild type PS1 transgenic mice, [¹¹C]-A uptake in the whole brain (cerebellum excluded) is higher than the uptake of [¹¹C]PK11195 (Figure 6). However, the uptake of both [¹¹C]-A and [¹¹C]PK11195 is not significantly higher in APP×PS1 vs wild type PS1. By contrast, specific of [³H]-A binding is about 2-fold greater in whole brain sections from APPxPS1 mice compared to wild type PS1 mice (Figure 7).
These data demonstrate that [¹¹C]-A can specifically detect increased PBR binding and inflammation in an acute model of rat neuroinflammation and in a mouse model of Alzheimer's disease. *In vivo* PET imaging confirms that [¹¹C]-A can be used to image PBR receptor overexpression and neuroinflammation in rodents. Moreover, the PBR receptor binding observed by PET imaging with [¹¹C]-A is greater than that observed with the reference PBR receptor PET ligand, [¹¹C]PK11195.

### EXAMPLE 8

The uptake of [¹¹C]-A uptake is significantly higher in the lesioned right striatum of a quinolinic acid-injected primate compared to [¹¹C]-A uptake in the contralateral non-injected striatum and two non-injected control brain regions (cerebellum, prefrontal cortex) 24 hrs after excitotoxin injection (Figure 8). [¹¹C]-A uptake in the contralateral non-injected striatum remained stable and at the same level as [¹¹C]-A uptake in the brain regions of a non-injected primate (Figure 8). The increase in [¹¹C]-A uptake could still be visualized at 48 hrs after excitotoxin injection (in the left hemisphere), whereas [¹¹C]-A uptake at 7 months after excitotoxin injection (kinetics in the right striatum lesioned 7 months earlier) had returned to baseline levels. A more detailed characterisation of the early phase of neuroinflammation demonstrates that the increased [¹¹C]-A uptake can be visualised from 24 hrs up to 16 days (Figure 9). Systemic administration of a large excess of unlabelled PK11195 30 min after radiotracer injection resulted in a specific displacement of the [¹¹C]-A binding in the lesioned striatum with no noticeable changes observed in the non-lesioned control brain regions (Figure 9).

### Applications:

The present invention can be applied as a diagnostic tool and as a tool to follow the evolution and progression of pathologies in which the levels of the PBR are altered and in which inflammation is present. The invention can also be applied to receptor occupancy studies and to evaluate the efficacy of therapeutic treatments in pathological conditions and as a translational biomarker for research from animal models to humans.

## Claims

1. Use of 7-chloro-N,N,5-trimethyl-4-oxo-3-phenyl-3,5-dihydro-4H-pyridazino[4,5-*b*]indole-1-acetamide as a biomarker for the detection, in an individual, of PBR levels associated with normal and pathological conditions.

2. Use according to claim 1 wherein 7-chloro-N,N,5-trimethyl-4-oxo-3-phenyl-3,5-dihydro-4H-pyridazino[4,5-*b*]indole-1-acetamide is radiolabelled.

3. Use according to claim 1 and 2 wherein 7-chloro-N,N,5-trimethyl-4-oxo-3-phenyl-3,5-dihydro-4H-pyridazino[4,5-*b*]indole-1-acetamide is radiolabelled with carbon-11.

4. Use according to claims 1 to 3 wherein 7-chloro-N,N,5-trimethyl-4-oxo-3-phenyl-3,5-dihydro-4H-pyridazino[4,5-*b*]indole-1-acetamide is radiolabelled with carbon-11 on the carbon of the methyl group located on position 5 of the indole nucleus.

5. Use according to claims 1 and 2 wherein 7-chloro-N,N,5-trimethyl-4-oxo-3-phenyl-3,5-dihydro-4H-pyridazino[4,5-*b*]indole-1-acetamide is radiolabelled with radiohalogens.

6. Use according to claims 1, 2 or 5 wherein 7-chloro-N,N,5-trimethyl-4-oxo-3-phenyl-3,5-dihydro-4H-pyridazino[4,5-*b*]indole-1-acetamide is preferentially radiolabelled with the radiohalogen fluorine-18.

7. Use according to claims 1-6 wherein the detection of PBR levels and inflammation is performed by PET imaging (positron emission tomography) or SPECT imaging (single photon emission computed tomography).

8. Use according to claims 1-6 wherein the detection of PBR levels and inflammation is performed by SPECT imaging (single photon emission computed tomography).

9. Use according to claims 1-8 wherein pathological conditions related to changes PBR levels are selected from brain injuries, brain infections and neurological diseases.

10. Use according to claims 1-8 wherein pathological conditions related to changes in PBR levels are selected from psychiatric diseases.

11. Use according to claims 1-8 wherein pathological conditions related to changes in PBR levels are selected from proliferative diseases.

12. Use according to claims 1-8 wherein pathological conditions related to changes in PBR levels are selected from peripheral inflammatory processes.

13. Use according to claims 1-8 wherein detection of PBR levels is performed for occupancy studies.

14. Use according to claims 1-8 wherein detection of PBR levels is performed for evaluating the efficacy of a therapeutic treatment.

15. Method for the detection of PBR levels associated with normal conditions and changes in PBR levels associated with pathological conditions wherein the detection is performed using a radiolabelled form of 7-chloro-N,N,5-trimethyl-4-oxo-3-phenyl-3,5-dihydro-4H-pyridazino[4,5-*b*]indole-1-acetamide.

16. Method according to claim 15 wherein the radiolabelled form of 7-chloro-N,N,5-trimethyl-4-oxo-3-phenyl-3,5-dihydro-4H-pyridazino[4,5-b]indole-1-acetamide contains carbon-11.

17. Method according to claims 15-16 wherein 7-chloro-N,N,5-trimethyl-4-oxo-3-phenyl-3,5-dihydro-4H-pyridazino[4,5-b]indole-1-acetamide is radiolabelled with carbon-11 on the carbon of the methyl group located on position 5 of the indole nucleus.

18. Method according to claims 15-17 wherein pathological conditions related to changes in PBR levels are selected from brain injuries, brain infections, and neurological diseases.

19. Method according to claims 15-17 wherein pathological conditions related to changes in PBR levels are selected from psychiatric diseases.

20. Method according to claims 15-17 wherein pathological conditions related to changes in PBR levels are selected from proliferative diseases.

21. Method according to claims 15-17 wherein pathological conditions related to changes in PBR levels are selected from peripheral inflammatory processes.

22. Method according to claims 15-17 wherein detection of PBR levels is performed for occupancy studies.

23. Method according to claims 15-17 wherein detection of PBR levels is performed for evaluating the efficacy of a therapeutic treatment.

24. Diagnostic kit for the detection of PBR levels associated with normal conditions and changes in PBR levels associated with pathological conditions comprising a radiolabelled form of 7-chloro-N,N,5-trimethyl-4-oxo-3-phenyl-3,5-dihydro-4H-pyridazino[4,5-b]indole-1-acetamide.
